# DEMANDE DE BREVET EUROPEEN

(11) **EP 4 527 428 A1**
(43) Date de publication de la demande: **26.03.2025**
(21) Numéro de dépôt: 23203000.7
(22) Date de dépôt: 11.10.2023
(51) Int. Cl.: A61M 5/158, A61M 5/32

(54) **AIGUILLE**

(30) Priorité: 19.09.2023 FR 2309922
(71) Demandeur: Seft Holding, 1950 Sion (CH)
(72) Inventeur: ZAMBAUX, Jean-Pascal, 33270 Bouliac (FR)
(74) Mandataire: Aquinov

(57) **Abrégé**

L'invention a pour objet une aiguille (1) comportant un corps cylindrique (10) s'étendant selon un axe longitudinal dont au moins une extrémité (100) est biseautée, ledit corps (10) étant formé au moins à partir d'un polymère, ladite aiguille (1) étant caractérisée en ce qu'elle comporte au moins un fil de renforcement (101) adapté pour rigidifier ledit corps (10), ledit fil de renforcement (101) étant formé au moins à partir d'un matériau à mémoire de forme.

## Description

### Domaine technique

L'invention se rapporte au domaine technique des aiguilles. L'invention concerne notamment une seringue comportant ladite aiguille.

### Etat de la technique

Les pyrogènes ou substances pyrogènes sont des impuretés qui, lorsqu'elles sont présentes dans un produit pharmaceutique destiné à être administré à un patient, provoquent une réaction fébrile lors de l'administration effective du produit pharmaceutique. Dans le domaine médical, de nombreux dispositifs comportent des aiguilles d'injection et/ou de perforation permettant l'écoulement d'un produit pharmaceutique, généralement un liquide. De telles aiguilles destinées à contenir et à transporter un produit pharmaceutique doivent non seulement être stériles et stérilisables, mais également apyrogènes, c'est-à-dire qu'elles ne contiennent pas de pyrogènes, et doivent être dépyrogénables, c'est-à-dire qu'elles doivent pouvoir subir un traitement qui élimine les éventuels pyrogènes qu'elles pourraient contenir. En effet, pour dépyrogéniser de telles aiguilles, il faut les chauffer jusqu'à 253°C pendant une heure. C'est l'une des raisons pour lesquelles les aiguilles pour l'injection d'un produit pharmaceutique dans les tissus humains sont généralement en métal. Une autre raison est que ces aiguilles pour injection dans les tissus humains doivent être très fines pour limiter la douleur ressentie par le patient, tout en conservant une résistance et une rigidité suffisantes pour la pénétration de la peau et des couches sous-cutanées. Cependant, les aiguilles métalliques, une fois utilisées, ne peuvent pas être éliminées facilement, ce qui pose un problème de santé sanitaire en raison des accidents de contamination résultant du contact avec ces aiguilles non stérilisées et non dépyrogénées. Pour résoudre ce problème d'aiguilles pour l'injection de produits pharmaceutiques, notamment dans le corps humain, des aiguilles en matière plastique ont été proposées.

En réalité, lorsqu'une aiguille en plastique à usage médical est employée, elle est à usage unique et non dépyrogénée, mais fabriquée dans des conditions telles qu'elle est apyrogène. Cependant, une fois sorti de son emballage et utilisé, il n'est pas dépyrogénable. De plus, ces aiguilles n'étant pas dépyrogénables, il n'est pas possible, même avant la première utilisation, de les dépyrogéniser pour être certain qu'elles sont bien apyrogènes. En effet, des problèmes de production ou des problèmes survenus lors de leur transport ou de leur manutention peuvent avoir entraîné une contamination par des pyrogènes.

On connait notamment des aiguilles à monter sur une seringue d'injection, qui soit à la fois dépyrogénable et facilement jetable. Ce type d'aiguille peut notamment être constituée d'un corps cylindrique qui s'étend selon un axe longitudinal dont au moins une extrémité est biseautée et réalisée en polymère polyaryléthercétone et renforcé par des fils en acier inoxydable. Toutefois, un inconvénient de ce type d'aiguilles est qu'elles sont rigides et peu adaptables.

L'invention se place donc dans ce contexte et cherche à résoudre l'ensemble des inconvénients précités. Ainsi, l'invention cherche à proposer une aiguille permettant d'éviter de se piquer en étant dépourvue d'inox mais dont le corps d'aiguille soit rigide.

### Présentation de l'invention.

L'invention a pour objet une aiguille comportant un corps cylindrique s'étendant selon un axe longitudinal dont au moins une extrémité est biseautée, ledit corps étant formé au moins à partir d'un polymère, ladite aiguille étant caractérisée en ce qu'elle comporte au moins un fil de renforcement adapté pour rigidifier ledit corps, ledit fil de renforcement étant formé au moins à partir d'un matériau à mémoire de forme.

Le corps cylindrique peut être creux. Le corps creux peut avoir une section circulaire. Le corps creux peut fournir un conduit pour l'écoulement, de préférence d'un liquide, notamment un liquide pharmaceutique à injecter ou à transfuser. Le corps de l'aiguille peut présenter au moins une extrémité biseautée pouvant permettre la perforation notamment des tissus humains. Dans un mode de réalisation dans lequel l'aiguille comporte une seule extrémité biseauté, l'autre extrémité peut être adaptée pour être insérée dans une seringue.

Dans un mode de réalisation, le corps peut comprendre au moins un fil de renforcement noyé dans le polymère dudit corps. Le fil peut s'étendre parallèlement à l'axe longitudinal sur toute la longueur du corps creux. Dans un mode de réalisation comportant plusieurs fils de renforcement, le corps peut comprendre trois fils de renforcement noyés dans le polymère polyaryléthercétone, s'étendant parallèlement à l'axe longitudinal X-X' sur toute la longueur du corps creux, étant équitendus et équidistants les uns des autres et répartis de sorte que les fils deux à deux définissent un angle au centre identique. Les trois fils de renfort peuvent être tendus uniformément sur toute leur longueur.

L'aiguille peut être adaptée pour l'injection dans les tissus humains et peut comporter un diamètre externe d'environ 0,7 mm et un diamètre interne d'environ 0,25 mm ce qui signifie par conséquent que l'épaisseur de la paroi de l'aiguille peut être d'environ 0,25 mm. A de telles épaisseurs de paroi, la rigidité, c'est-à-dire la résistance à la déformation peut être insuffisante. L'aiguille peut présenter une épaisseur faible telle que mentionnée ci-dessus pour permettre de minimiser la douleur et les lésions tissulaires pour le patient.

Le polymère peut être du polyaryléthercétone, du polyéthylène, du polycarbonate ou du polypropylène. Le polymère peut comprendre des charges choisies parmi les fibres de carbone, les fibres de verre, les granulés de graphite, les granulés de polytétrafluo-roéthylène (PTFE), les granulés de carbone noir et les mélanges de deux ou plusieurs de ceux-ci.

Le fil peut comporter un diamètre inférieur à 500µm, notamment inférieur à 300 µm. Au vu de ce diamètre, lorsque l'aiguille est exposée à une flamme ou à un chauffage à haute température, afin de la rendre inutilisable et d'éviter la possibilité de perforation à partir de son extrémité biseautée, les fils peuvent également fondre et la sécurité sanitaire de l'aiguille peut être conservée.

Le matériau à mémoire de forme peut être souple et/ou élastique pour une première plage de température et peut être rigide pour une deuxième plage de température.

Avantageusement, le polymère est un oxy-1,4-phénylène-oxy-1,4-phénylène-carbonyl-1,4-phénylène.

Le polymère peut être commercialisé sous le nom de PEEK^{®} (marque déposée). Le polymère peut être un polymère linéaire aromatique semi-cristallin. Le polymère peut comporter une température de fusion de 343°. Le polymère peut comporter une température de transition vitreuse est de 143°C. Le polymère peut comporter une température de fonctionnement d'environ 260°C. Le polymère peut comporter une température de ramollissement pouvant atteindre 315°C. L'aiguille comportant le polymère peut être dépyrogénable, c'est-à-dire qu'elle peut être chauffée à environ 253°C pendant notamment une heure sans subir de déformation ou d'altération de ses propriétés chimiques et mécaniques. Le polymère peut être adapté pour supporter une étape ultérieure d'extrusion. Le coefficient de dilatation du polymère est sensiblement le même que celui de l'inox donc il peut se solidifier en chauffant pour être rigidifié et garde la même forme après rigidification.

Le polymère peut être biocompatible avec les tissus humains. Le polymère peut être intrinsèquement pur, avec de très faibles niveaux de libération de gaz et d'ions extractibles.

Avantageusement, le fil de renforcement présente au moins une portion en forme de spirale autour dudit axe longitudinal.

Le fil de renforcement peut être noyé dans le polymère polyaryléthercétone en s'étendant autour d'un axe de rotation confondu avec l'axe longitudinal sur toute la longueur du corps creux. Dans le mode de réalisation comportant trois fils, lesdits fils peuvent être agencés de sorte que chacun s'étende autour d'un axe de rotation confondu avec l'axe longitudinal sur toute la longueur du corps creux, lesdits fils ayant un sens de rotation identique. Les trois fils peuvent être à égale distance les uns des autres.

Avantageusement, l'aiguille comporte une pluralité de fils de renforcement formant un maillage.

Dans ce mode de réalisation, la pluralité de fils s'étend autour de l'axe longitudinal sur toute la longueur du corps creux, de sorte que lesdits fils s'entrelacent pour former un maillage. Les fils peuvent se chevaucher et se croiser.

Avantageusement, le fil comporte une portion rigidifiée et une portion souple.

C'est-à-dire que le fil peut sur toute sa longueur comporter un même matériau mais avec une partie rigidifiée et une partir non rigidifiée. Dans un autre mode de réalisation, le fil peut comporter deux matériaux, un premier matériau formant une portion rigidifiée et un deuxième matériau formant une portion non rigidifiée. Une aiguille peut ainsi comporter une portion souple et une portion rigide ou semi rigide.

Avantageusement, chaque fil est de section circulaire.

Dans ce mode de réalisation, chaque fil peut comporter un diamètre d'environ 0,1 mm.

Avantageusement, chaque fil est de section elliptique.

Dans ce mode de réalisation, chaque fil peut comporter axe principal est d'environ 0,15 mm.

Avantageusement, chaque fil est de section triangulaire.

Le fil de section triangulaire peut être avantageusement utilisé puisqu'il peut permettre d'être plus rigide qu'un fil de section circulaire.

Avantageusement, le fil de renforcement est agencé dans le corps de l'aiguille.

Dans un autre mode de réalisation, le fil peut former un cylindre à mémoire de forme, c'est-à-dire que le fil peut former un cylindre creux positionné autour de l'aiguille et compris dans la lumière du corps creux. Dans ce mode de réalisation, l'aiguille peut être jetable et le cylindre à mémoire de forme réutilisable, la partie rigidifiée à mémoire de forme évite donc d'être en contact avec le produit.

Avantageusement, le corps de ladite aiguille est obtenu selon un procédé comportant une première étape du polymère de polyaryléthercétone simultanément à une étape de pultrusion du au moins un fil de renforcement au sein dudit polymère de polyaryléthercétone extrudé.

L'extrusion du polymère peut être simultanée à l'insertion du fil dans le canal extrudé. L'étape de pultrusion peut correspondre à une étape d'insertion du fil.

Avantageusement, ledit au moins un fil de renforcement est rigidifié, simultanément à l'étape de pultrusion.

Le fil de renforcement peut se rigidifier à une température supérieure à 80°. Dans un mode de réalisation, le fil peut se rigidifier à une température adaptée pour la stérilisation.

La rigidification du fil de renforcement peut se faire par chauffage dudit fil, une fois que le fil a atteint sa température de rigidification, celui-ci peut prendre sa forme et lors du refroidissement conserver cette forme.

Une fois que le fil est rigidifié, la stérilisation n'a pas d'impact sur la rigidité du corps.

Dans un mode de réalisation, le corps de l'aiguille est obtenu selon un procédé comportant une première étape d'extrusion du polymère de polyaryléthercétone pour former un corps pourvu d'un canal et d'une deuxième étape d'insertion du au moins un fil dans ledit canal du corps. Dans ce mode de réalisation, l'insertion du fil peut être réalisée dès la fin de l'extrusion pour éviter que la chauffe du fil ne fasse fondre le polymère et ainsi éviter que le fil soit en contact avec la lumière du corps de l'aiguille et évite qu'il puisse contaminer les produits à injecter.

Dans un autre mode de réalisation, le au moins un fil de renforcement est rigidifié, ultérieurement à l'étape de pultrusion, dans une étape de stérilisation par chaleur sèche dans laquelle le corps de ladite aiguille incorporant le fil de renforcement est chauffé à une température d'au moins 160°.

Avantageusement, ledit au moins un fil de renforcement est rigidifié, ultérieurement à l'étape de pultrusion, dans une étape de stérilisation par chaleur humide dans laquelle le corps de ladite aiguille incorporant le fil de renforcement est chauffé à une température d'au moins 121°.

Le fil de renforcement peut être chauffé en chaleur sèche ou en chaleur humide.

Avantageusement, le corps de ladite aiguille est obtenu selon un procédé comportant une première étape d'impression additive du corps à partir d'une bobine de fil de polymère de polyaryléthercétone autour du au moins un fil de renforcement.

Le corps cylindrique peut comporter une section transversale circulaire qui peut permettre de minimiser la douleur lors de l'insertion de l'aiguille dans les tissus humains.

Avantageusement, le corps de ladite aiguille est obtenu selon procédé comportant une première étape d'impression soustractive du corps dans un bloc de polymère de polyaryléthercétone dans lequel est agencé le au moins un fil de renforcement.

Avantageusement, le fil de renforcement est agencé autour du corps de l'aiguille.

Dans ce mode de réalisation, le fil peut former un fourreau comportant une lumière et le polymère formant le corps de l'aiguille peut être inséré dans ladite lumière.

Avantageusement, le corps de ladite aiguille est obtenu selon un premier procédé, et le fil de renforcement est obtenu selon un deuxième procédé pour former un fourreau dans lequel est agencée le corps de l'aiguille.

Avantageusement, le au moins un fil est en matériau composite.

Le matériau peut être un alliage à mémoire de forme (AMF ou en anglais SMA pour shape memory alloy) notamment à base de cuivre (par exemple de formule chimique CuAIBe, CuAINi, CuAIMn, CuZnAI), à base de nickel-titane (par exemple de formule chimique NiTi, NiTiFe, NiTiCu, NiTiCr, NiTiHf, NiTiCo, NiTiNb etc.), et les alliages à base de fer (par exemple de formule chimique FeMnSi, FeMnCr, FeMnCrSi). Certains de ces alliages à mémoire de forme peuvent être monocristallins, c'est-à-dire constitués d'un seul grain, ou de plusieurs grains séparés par des joints de grain à faible désorientation.

La température d'activation du matériau composite lors de la chauffe, c'est-à-dire dans la transition de la phase martensite vers la phase austénite, peut être comprise entre 45°C et 450°C.

Un autre aspect de l'invention concerne une seringue d'injection comportant un piston ; un corps de pompe équipé d'un raccord terminal d'ajustement d'une aiguille d'injection ; une aiguille d'injection selon l'une des quelconques revendications précédentes.

La seringue peut être adaptée pour l'injection de produits pharmaceutiques, notamment dans les tissus humains. La seringue peut comporter un corps de pompe faisant office de réservoir pour un produit pharmaceutique à injecter. Le corps de pompe peut comporter un raccord terminal comportant un embout adapté pour recevoir l'aiguille d'injection. Le corps de pompe peut comporter un piston agencé dans ledit corps de pompe, adapté pour propulser le produit pharmaceutique à travers l'aiguille.

L'aiguille et la seringue de l'invention peuvent être réalisés par tout procédé connu de l'homme du métier, tel qu'un procédé de moulage par injection, un procédé d'extrusion ou un procédé d'extrusion sous tension dit procédé de pultrusion dans lequel les fils de renfort sont maintenus sous tension lors de leur passage, et celle de la matrice polymère, dans la filière d'extrusion.

L'aiguille, la seringue et le connecteur de l'invention peuvent être réalisés par tout procédé connu de l'homme du métier, tel qu'un procédé de moulage par injection, un procédé d'extrusion ou un procédé d'extrusion sous tension dit procédé de pultrusion dans lequel les fils de renfort sont maintenus sous tension lors de leur passage dans la filière d'extrusion.

Avantageusement, le corps de pompe équipé de son raccord terminal et le piston sont en un polymère polyaryléthercétone.

La seringue peut être réalisée en un polymère polyaryléthercétone, ce qui signifie que le piston et le corps de pompe équipé de son embout peuvent être entièrement réalisés en un tel polymère.

Avantageusement, le corps de pompe équipé de son raccord terminal et le piston sont en un polymère polyaryléthercétone comprenant des charges choisies parmi les fibres de verre, les fibres de carbone, les granules de graphite, les granules de polytétrafluo-roéthylène (PTFE), les granules de noir de carbone et les mélanges de deux ou plusieurs d'entre eux.

### Brève description des figures.

D'autres avantages et caractéristiques de la présente invention sont maintenant décrits à l'aide d'exemples uniquement illustratifs et nullement limitatifs de la portée de l'invention, et à partir des dessins annexés, dessins sur lesquels les différentes figures représentent :
[Fig. 1] représente une vue partielle en coupe longitudinale de l'extrémité d'une aiguille selon un mode de réalisation de l'invention.
[Fig.2] représente une vue partielle longitudinale de l'extrémité d'une aiguille selon un autre mode de réalisation de l'invention.
[Fig.3] représente une vue partielle longitudinale de l'extrémité d'une aiguille selon un mode différent de réalisation de l'invention.
[Fig.4] représente une vue en coupe transversale d'une aiguille selon un mode de réalisation.
[Fig.5] représente une vue partielle en coupe longitudinale d'une seringue comportant une aiguille selon l'invention.

Dans la description qui suit, les éléments identiques, par structure ou par fonction, apparaissant sur différentes figures conservent, sauf précision contraire, les mêmes références.

### Description d'un mode de réalisation.

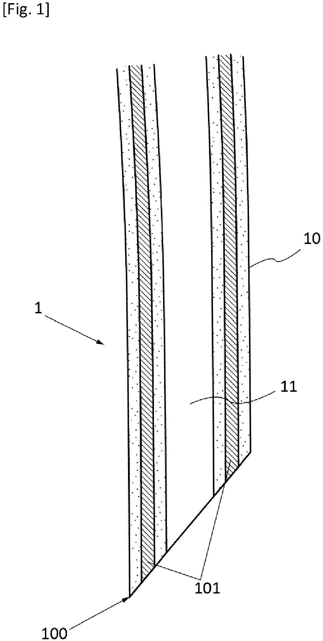

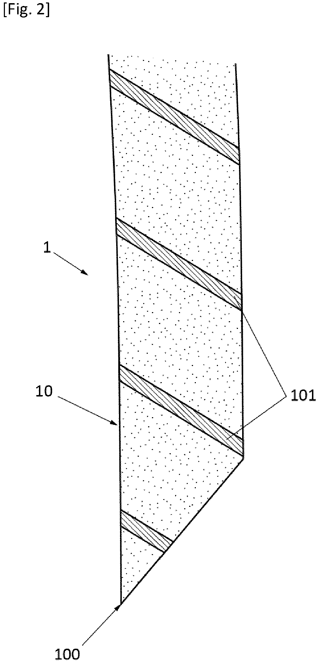

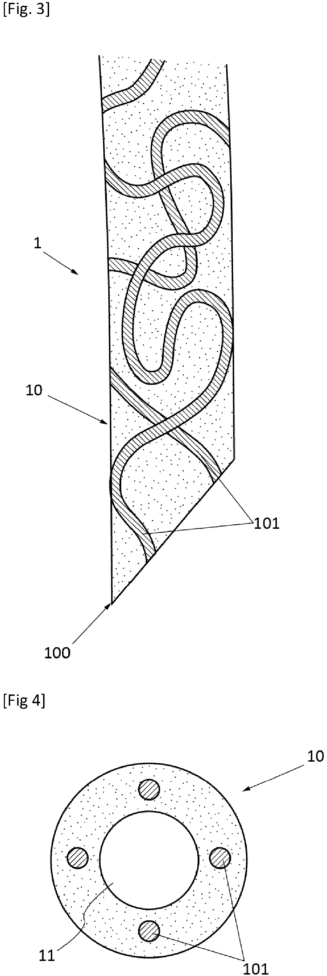

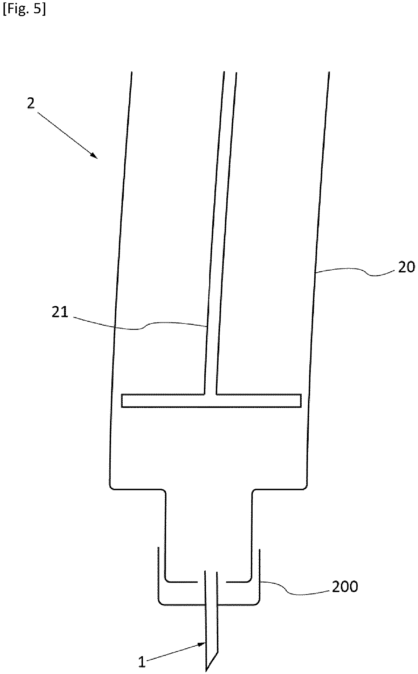

On a représenté en [Fig. 1] une vue partielle en coupe longitudinale de l'extrémité d'une aiguille selon un mode de réalisation de l'invention. L'aiguille est également décrite en lien avec les [Fig.2] et [Fig.3] représentant chacune différents modes de réalisation de l'aiguille et avec la [Fig.4] représentant une vue en coupe transversale d'une aiguille selon un mode de réalisation. L'aiguille est également décrite en [Fig.5], dans un mode de réalisation dans lequel l'aiguille est utilisée dans une seringue.

L'aiguille 1 comporte un corps cylindrique 10 s'étendant selon un axe longitudinal. L'aiguille 1 comporte au moins une extrémité biseautée 100. L'extrémité biseautée 100 est adaptée pour la perforation des tissus humains. Le corps cylindrique 10 est creux et comporte en son centre une lumière 11. Le corps 10 a une section circulaire. Le corps 10 comporte au moins un polymère. Le polymère est un oxy-1,4-phénylène-oxy-1,4-phénylène-carbonyl-1,4-phénylène. Le polymère se solidifie en chauffant pour être rigidifié et garde la même forme après rigidification.

L'aiguille 1 comporte au moins un fil de renforcement 101 adapté pour rigidifier le corps. Le fil de renforcement 101 comporte un matériau à mémoire de forme. Le matériau peut être un alliage à mémoire de forme. Le fil de renforcement est noyé dans le polymère du corps. Chaque fil de renforcement comporte un diamètre de 0,1 mm.

Dans le mode de réalisation décrit en [Fig.1], le corps 10 comprend trois fils 101 s'étendant parallèlement à l'axe longitudinal sur toute la longueur du corps 10. Les fils 101 sont équitendus et équidistants les uns des autres et répartis de sorte que les fils 101 deux à deux définissent un angle au centre identique.

Dans le mode de réalisation décrit en [Fig.2], le corps 10 comprend quatre fils 101 s'étendant autour du corps 10 de l'aiguille 1. Les fils de renforcement 101 présentent au moins une portion en forme de spirale s'étendant autour de l'axe longitudinal du corps. Les fils 101 ont un sens de rotation identique. Les fils 101 sont à égale distance les uns des autres. Le fil de renforcement 101 peut être noyé dans le polymère en s'étendant autour d'un axe de rotation confondu avec l'axe longitudinal sur toute la longueur du corps 10.

Dans le mode de réalisation décrit en [Fig.3], le corps 10 comprend une pluralité de fils 101 formant un maillage. La pluralité de fils 101 s'étend autour de l'axe longitudinal sur toute la longueur du corps 10, de sorte que lesdits fils 101 s'entrelacent pour former un maillage. Les fils 101 se chevauchent et se croisent.

Dans le mode de réalisation décrit à la [Fig.4], chaque fil 101 a une section circulaire et l'aiguille 1 comporte quatre fils de renforcement 101.

La [Fig.5] décrit une seringue d'injection 2 comportant un piston 21, un corps de pompe 20 équipé de l'aiguille 1.

Le corps de pompe 20 fait office de réservoir pour un produit pharmaceutique à injecter. Le corps de pompe 20 comporte un raccord terminal 200 comportant un embout adapté pour recevoir l'aiguille d'injection 1. Le corps de pompe 20 comporte un piston 21 agencé dans ledit corps de pompe 20, adapté pour propulser le produit pharmaceutique à travers l'aiguille 1.

Le corps de pompe 20 équipé de son raccord terminal 200 et le piston 21 sont en polymère. Le corps de pompe 20, le raccord terminal 200 et le piston 21 sont en polymère polyaryléthercétone.

La description qui précède explique clairement comment l'invention permet d'atteindre les objectifs qu'elle s'est fixée, à savoir proposer une aiguille permettant d'éviter de se piquer en étant dépourvue d'inox mais dont le corps d'aiguille soit rigide, en proposant une aiguille comportant un corps cylindrique s'étendant selon un axe longitudinal dont au moins une extrémité est biseautée, ledit corps étant formé au moins à partir d'un polymère de polyaryléthercétone, ladite aiguille étant caractérisée en ce qu'elle comporte au moins un fil de renforcement adapté pour rigidifier ledit corps, ledit fil de renforcement étant formé au moins à partir d'un matériau à mémoire de forme.

En tout état de cause, l'invention ne saurait se limiter aux modes de réalisation spécifiquement décrits dans ce document, et s'étend en particulier à tous moyens équivalents et à toute combinaison techniquement opérante de ces moyens. On pourra en particulier envisager :
- Chaque fil est de section elliptique ;
- Chaque fil est de section triangulaire ;
- Le corps cylindrique est biseauté aux deux extrémités ;
- Le fil de renforcement est agencé dans le corps de l'aiguille ;
- Le fil peut former un cylindre à mémoire de forme c'est-à-dire que le fil peut former un cylindre creux positionné autour de l'aiguille et compris dans la lumière du corps cylindrique ;
- Le fil peut être agencé autour du corps de l'aiguille et former un fourreau comportant une lumière et le polymère formant le corps de l'aiguille peut être inséré dans ladite lumière.

## Revendications

1. Aiguille (1) comportant un corps cylindrique (10) s'étendant selon un axe longitudinal dont au moins une extrémité (100) est biseautée, ledit corps (10) étant formé au moins à partir d'un polymère, ladite aiguille (1) étant **caractérisée en ce qu'**elle comporte au moins un fil de renforcement (101) adapté pour rigidifier ledit corps (10), ledit fil de renforcement (101) étant formé au moins à partir d'un matériau à mémoire de forme.

2. Aiguille (1) selon la revendication précédente **caractérisée en ce que** le polymère est un oxy-1,4-phénylène-oxy-1,4-phénylène-carbonyl-1,4-phénylène.

3. Aiguille (1) selon l'une des revendications précédentes **caractérisée en ce que** le fil de renforcement (101) présente au moins une portion en forme de spirale autour dudit axe longitudinal.

4. Aiguille (1) selon l'une des revendications précédentes **caractérisée en ce qu'**elle comporte une pluralité de fils de renforcement (101) formant un maillage.

5. Aiguille (1) selon l'une des revendications précédentes **caractérisée en ce que** le fil (101) comporte une portion rigidifiée et une portion souple.

6. Aiguille (1) selon l'une des revendications précédentes, **caractérisé en ce que** le fil de renforcement (101) est agencé dans le corps (10) de l'aiguille.

7. Aiguille (1) selon la revendication 6, **caractérisée en ce que** le corps (10) de ladite aiguille (1) est obtenu selon un procédé comportant une première étape d'extrusion du polymère de polyaryléthercétone simultanément à une étape de pultrusion du au moins un fil de renforcement (101) au sein dudit polymère de polyaryléthercétone extrudé.

8. Aiguille (1) selon la revendication 6, **caractérisée en ce que** le corps de ladite aiguille (1) est obtenu selon un procédé comportant une première étape d'impression additive du corps (10) à partir d'une bobine de fil de polymère de polyaryléthercétone autour du au moins un fil de renforcement (101).

9. Aiguille (1) selon la revendication 6, **caractérisée en ce que** le corps (10) de ladite aiguille (1) est obtenu selon procédé comportant une première étape d'impression soustractive du corps (10) dans un bloc de polymère de polyaryléthercétone dans lequel est agencé le au moins un fil de renforcement (101).

10. Seringue d'injection (2) comportant :
- Un piston (21) ;
- Un corps de pompe (20) équipé d'un raccord terminal (200) d'ajustement d'une aiguille d'injection (1) ;
- Une aiguille (1) d'injection selon l'une des quelconques revendications précédentes.
